# EUROPEAN PATENT APPLICATION

(11) **EP 2 548 601 A1**
(43) Date of publication of application: **23.01.2013**
(21) Application number: 12177026.7
(22) Date of filing: 19.07.2012
(51) Int. Cl.: A61M 25/00, A61B 17/3207

(54) **An aspiration catheter and related method**

(30) Priority: 22.07.2011 IT TO20110661
(71) Applicant: CID S.p.A., 13040 Saluggia (VC) (IT)
(72) Inventor: Vallana, Franco, I-13040 Saluggia (VC) (IT); Curcio, Maria, I-13040 Saluggia (VC) (IT); Gard, Marco, I-13040 Saluggia (VC) (IT)
(74) Representative: Bosotti, Luciano

(57) **Abstract**

A catheter for aspirating occlusive formations (C) in a patient's body includes an aspiration duct (14) with a distal suction opening (16). The aspiration duct (14) is to be brought to a subatmospheric pressure level (S) to aspirate an occlusive formation (C) through said distal suction opening (16). An unblocking duct (22) is provided to feed to the distal portion of aspiration duct (14) a pressurized fluid (S), to act in a proximal-distal direction on a block (B) formed in said aspiration duct (14). An occluder element (20) acts on said distal suction opening (16) to prevent the escape of the pressurized fluid from distal suction opening (16).

## Description

### Technical field

The present description relates to aspiration catheters.

In various embodiments, the description may relate to catheters for aspirating thrombi or, in general, occlusive formations in the vascular system.

### Technological background

Aspiration catheters, for example catheters for aspirating thrombi, normally comprise a thin tube adapted to be introduced, by catheterism, into the vascular system of a patient, so as to bring the catheter distal opening in the vicinity of an occlusive formation to be removed. By inducing a subatmospheric pressure in the inner cavity of the catheter (which can be filled for example with a liquid, e.g. physiological solution), the occlusive formation can be aspirated within the catheter, resulting in the removal thereof.

During the use of such catheters, the aspirated material within the catheter can occlude the cavity of the catheter itself, therefore blocking the suction mechanism. If it is not possible to restore the patency of the catheter by increasing the suction intensity, in order to complete the removal of the occlusive formation it is necessary to take out that catheter and to introduce a new one; in some cases, in order to complete the removal of the occlusive formation, it may be necessary to repeat such a removal and substitution several times.

This makes the procedure more complex and its execution takes a longer time.

### Object and summary

The object of the present invention is to overcome the previously outlined drawbacks.

According to the invention, such an object is achieved by an aspiration catheter having the features specifically set forth in the claims that follow. The invention also concerns a related method.

The claims are an integral part of the technical teaching of the invention provided herein.

Various embodiments may base their operation on the fact that, if it is attempted to unblock the catheter by increasing the suction intensity (and therefore raising the "vacuum" level inside the catheter), the pressure acting on the material causing the block cannot be higher than one atmosphere: this condition corresponds to a virtual situation wherein it is possible to create, in the catheter portion arranged proximally of the block, an absolute vacuum.

Various embodiments may base their operation on the principle of creating, in the catheter portion arranged distally of the site where the catheter is blocked, a pressure level higher than one atmosphere (even amounting to several atmospheres), being therefore able to exert a more effective removing function on the block present within the catheter.

### Brief description of the Figures

The invention will now be described, by way of non-limiting example only, with reference to the enclosed views, wherein:
- Figure 1 shows the overall structure of an embodiment, and
- Figures 2 and 3 are enlarged axial section views of the distal portion of an embodiment, shown in two possible operating conditions.

### Detailed description

In the following description, numerous specific details are given to provide a thorough understanding of embodiments. The embodiments can be practiced without one or several specific details, or with other methods, components, materials, etc. In other instances, well-known structures, materials, or operations are not shown or described in detail to avoid obscuring aspects of the embodiments.

Reference throughout this specification to "one embodiment" or "an embodiment" means that a particular feature, structure, or characteristic described in connection with the embodiment is included in at least one embodiment. Thus, the appearances of the phrases "in one embodiment" or "in an embodiment" in various places throughout this specification are not necessarily all referring to the same embodiment. Furthermore, the particular features, structures, or characteristics may be combined in any suitable manner in one or more embodiments.

The headings provided herein are for convenience only and do not interpret the scope or meaning of the embodiments.

In the Figures, reference 10 denotes on the whole an aspiration catheter.

In various embodiments, catheter 10 may be a tubular structure of a material commonly used for a catheter of this kind, such as for example polyamide, polyimide, etc., possibly reinforced with metal materials, high modulus fibres, etc.

In various embodiments, catheter 10 may be comprised of a distal portion, denoted with 10a, and a proximal portion, denoted with 10b.

As used in the present description, the terms "distal" and "proximal" refer to the insertion direction of catheter 10.

Catheter 10 is adapted to be introduced into a patient's vascular system, starting from end 10a which is brought to a site where an occlusive formation C (for example a thrombus in a blood vessel) is to be removed, with the possibility to manoeuvre catheter 10 from the proximal end 10b thereof, which remains outside the patient's body.

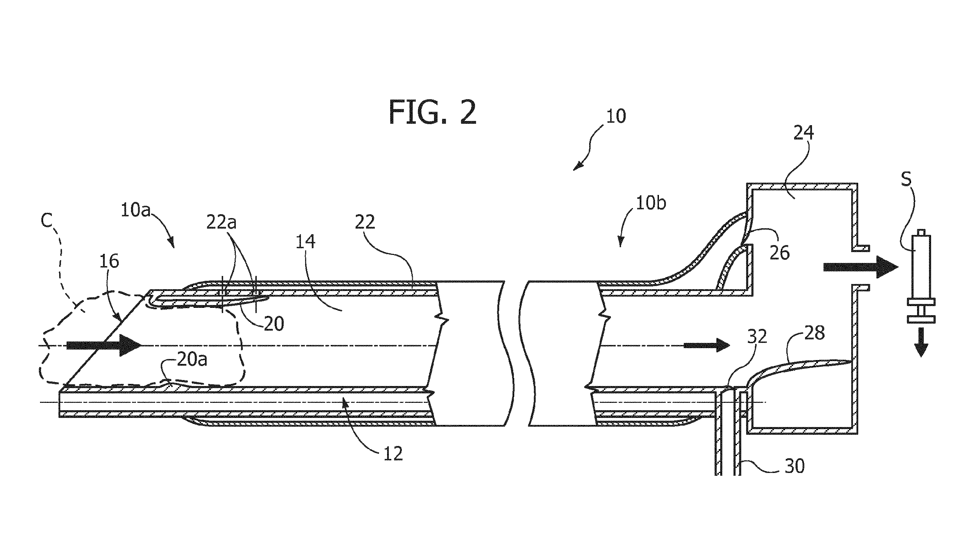

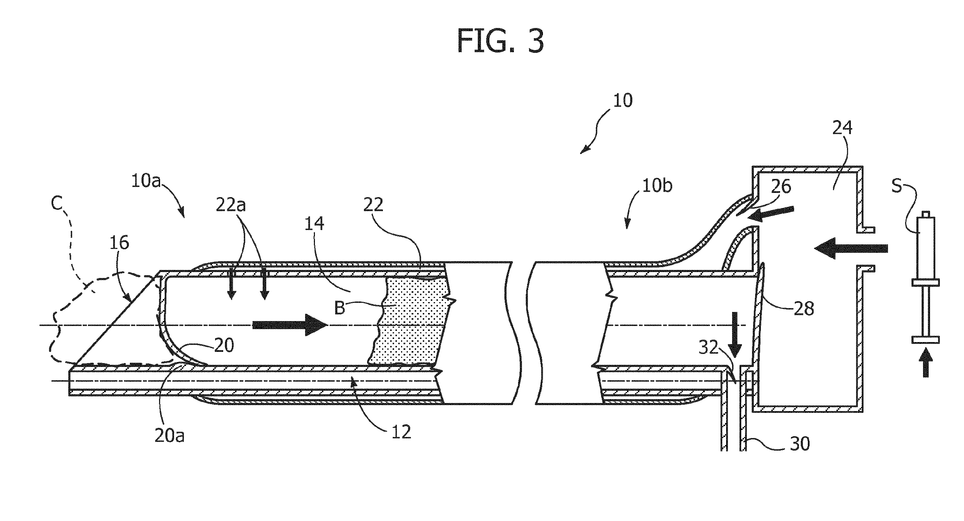

In various embodiments, catheter 10 may be introduced into the patient's body by sliding it along a guide wire W, inserted into a guide structure such as a tubular passageway 12, which is coupled to catheter 10: in this respect, see for example the section views of Figures 2 and 3.

In various embodiments, catheter 10 may have a generally tubular structure, with a cavity or duct 14 extending along catheter 10 and debouching at distal end 10a into a suction opening 16.

In various embodiments, distal end 10a may have a chamfered shape. Such a shape may ease the introduction and the advancement of the catheter towards the treatment site, through the meandering of the vascular system, and can moreover obtain a suction opening 16 with broader size as compared to the net cross section of cavity 14.

At the catheter proximal end 10a, cavity 14 may be adapted to be connected (for example via a luer-lock connector 18) to a suction device (i.e. to a source of subatmospheric pressure). Such a source of suction /subatmospheric pressure may simply be comprised of a syringe S1, adapted to be controlled by the operator performing the removal of the occlusive formation, so as to bring inner cavity 14 of the catheter (which may be filled with a liquid such as physiological solution) to a subatmospheric pressure level ("vacuum").

The implementation criteria for an aspiration catheter 10 according to the previously described principle, and the criteria adopted for the insertion thereof into a patient's body, in order to perform a removal operation by suction of an occlusive formation C, are to be held as well known in the art, and therefore do not require a detailed description herein.

In various embodiments, at distal end 10a, for example closely adjoining end opening 16 of axial cavity 14 of the catheter, there may be provided an occluder element 20, performing the functions of:
- making it possible to aspirate, into cavity 14, occlusive formation C (a thrombus or the like) which must be removed from the patient, according to the previously described procedure; and/or
- preventing an escape, for example of liquids, outside the catheter when, in ways to be better described in the following, the distal portion of catheter 10 is pressurized, i.e. brought to a pressure higher than atmosphere.

In various embodiments, occluder element 20 may be comprised, for example, of a valve having one or more flaps or leaflets, substantially corresponding to currently used structures for heart valve prostheses with one or more flaps. Such valves, adapted to be brought to closure by the pressurized fluid which is fed (as will be better detailed in the following) to the distal portion of catheter 10, can be made of plastic material, for example polyurethane (see for instance documents such as US-A-4 222 126 or US-A-4 265 694).

In various embodiments it is possible to employ valves adapted to be controlled for opening and/or closing, for example by resorting to shape memory materials which change their structure as a consequence of a temperature variation induced e.g. by a thermoelectric effect.

In various embodiments, a valve may be used which is made of a piece with the body of catheter 10.

Figures 2 and 3 schematically show the possible behaviour of such an occluder element.

Specifically:
- Figure 2 refers to a condition wherein cavity 14 of the catheter is brought to a subatmospheric pressure value (for example because syringe S1 is controlled for suction) in order to allow the aspiration, within cavity 14, of occlusive formation C which must be removed from the patient: in such a state, valve 20 is open, the valve leaflet or leaflets being kept displaced laterally from cavity 14 by the pressure, and therefore keeping end opening 16 opened;
- Figure 3 shows on the contrary a state wherein in the distal portion of cavity 14 a superatmospheric pressure is present (obtained according to the criteria and for the purposes better detailed in the following): in this state, valve 20 is closed, the valve leaflet or leaflets being urged by the pressure to extend through cavity 14 (for example abutting with complementary formations 20a provided on the wall of cavity 14), so as to occlude end opening 16.

In various embodiments, occluder element 20 may be implemented with different means from a flap or leaflet valve, for example as a balloon as used in balloon catheters, wherein:
- when cavity 14 is brought to a subatmospheric pressure level, the balloon is kept deflated, enabling the suction of occlusive formation C into cavity 14, through end opening 16;
- when the distal portion of cavity 14 is brought to a superatmopheric pressure level, the balloon is inflated, so as to occlude end opening 16.

Various embodiments of occluder element 20 may employ structures of devices used to selectively occlude a blood vessel.

In various embodiments, catheter 10 may be formed, totally or in part, as a double walled structure or, in general, as a structure that creates an "unblock" duct (for example a flow duct 22 of annular section, visible in Figures 2 and 3) so as to let a pressurized fluid (for example, in this case as well, physiological solution) flow towards distal portion 10a of catheter 10.

In various embodiments, such a pressurized fluid may be pumped into duct 22 starting from proximal end 10b of catheter 10 via a pumping device.

Once again, in various embodiments, such a pumping device may be for example a syringe S2, adapted to be used, in the same way as syringe S1, by the operator performing the aspiration procedure.

In various embodiments, syringe S2 may be coupled to duct 22 through a connector 24, for example a luer lock connector, by coupling onto a Y branching of proximal portion 10b of the catheter: such connections are known for instance in catheters with plural cavities.

In various embodiments, the pumping devices which in the present case are exemplified as discrete members, in the form of syringes S1 and S2 (the former being adapted to be used mainly for aspiration on axial cavity 14, in order to aspirate occlusive formation C into the catheter; the latter being adapted to be used mainly for delivery to duct 22, in order to pressurize the distal portion of catheter 10 according to criteria better detailed in the following) may be replaced by a single pumping member (for example a single syringe) adapted to work alternatingly as a suction source (subatmospheric pressure), to act on cavity 14, and as a delivery source (superatmospheric pressure), to act on duct 22.

In this regard, Figures 2 and 3 refer to the possibility of employing one single syringe S, connected to cavity 14 and to duct 22 through a distributing module 24 with two valves 26 and 28.

For simplicity of illustration, for valves 26 and 28 a symbolic representation has been used, similar to the one adopted for the valve adapted to be used as an occluder element 20: in various embodiments, distributing module 24 may be implemented as a directional spool valve, of the kind commonly used in hydraulic systems.

In various embodiments, the function of module 24 may be as follows:
- when syringe S (or a similar source) is operated for suction, it is contacted with cavity or duct 14 and isolated from duct 22 (Figure 2),
- when the syringe / the source is operated for delivery, it is contacted with duct 22 and isolated from cavity 14 (Figure 3).

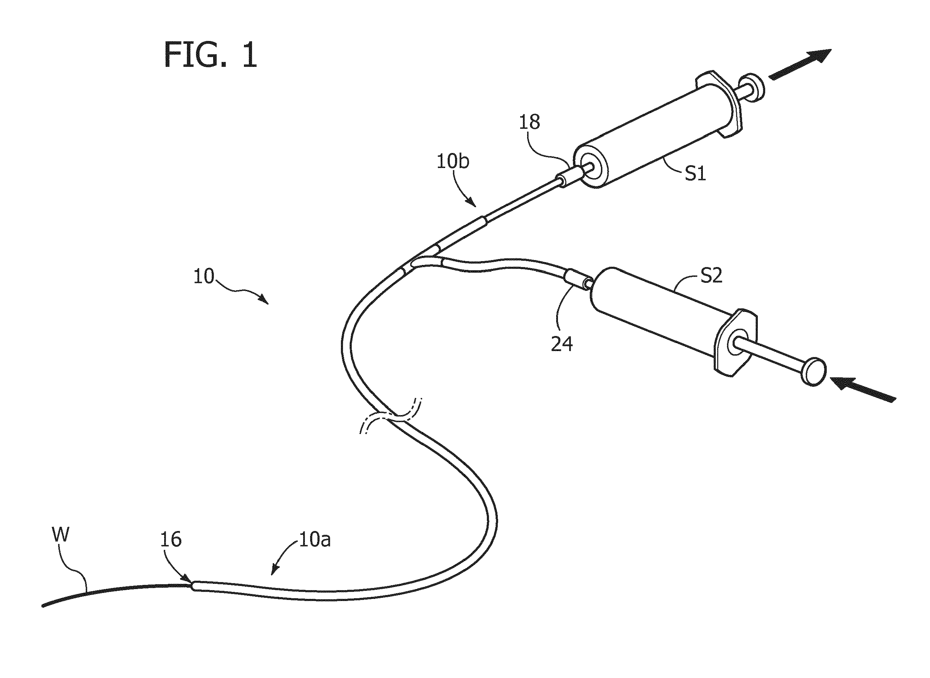

Said suction and delivery action on both ducts 14 and 22 (be it performed with two discrete elements S1 and S2, Figure 1, or be it performed with one single element S, which operates alternatingly as a suction element and as a delivery element, Figures 2 and 3) may be performed in both ducts 14 and 22 by using a physiological solution as a suction/delivery fluid.

In various embodiments, catheter 10 may be used according to criteria described in the following.

Catheter 10 is introduced into the patient's vascular system according to a conventional procedure, by bringing end opening 16 to the site where the suction of an occlusive formation C (for example a thrombus in a coronary artery) is to be performed.

According to the conventional procedure, cavity 14 of the catheter is brought to a subatmospheric pressure level (for example by operating syringe S1 of Figure 1, or else operating syringe S as exemplified in Figure 2), so as to perform a suction operation with the aim of aspirating occlusive formation C into cavity 14.

Said suction operation may go on smoothly until occlusive formation C is totally aspirated into catheter 10, and therefore removed from the vascular site where it was previously located.

In this case, catheter 10 which now contains occlusive formation C is simply extracted from the patient, in this way ending the aspiration procedure.

If during the performance of the procedure it is determined that the material aspirated into cavity 14 has caused an undesirable block of the cavity itself (as schematically shown with B in Figure 3), therefore preventing the continuation of the suction action until a complete removal of occlusive formation C, the operator may act (for example by using syringe S2 in Figure 1 or else by using syringe S in the structure shown in Figure 3) so as to lead into cavity 14, through one or several openings 22a present at the distal end of catheter 10, the pressurized fluid (for example a physiological solution) fed through duct 22.

Such pressurized fluid (which can reach a pressure level as high as several atmospheres, thanks to a positive action of delivery pumping) does not escape from end opening 16.

Actually, opening 16 is closed by occluder element 20: this may be for example the valve shown in Figures 2 and 3, the flaps or leaflets whereof can reach, thanks to the fluid pressure, the closed position of Figure 3; or it may be the aforementioned balloon, which is inflated so as to block opening 16.

The pressurized fluid fed into cavity 14 through duct 22 acts therefore between element 20 and block B which has formed in the catheter, in a distal position with respect to such block B, irrespective of the position in the catheter where block B has formed.

The action of such pressurized fluid (which, as previously stated, may reach a pressure level of several atmospheres, thanks to a positive action of delivery pumping) may exert a strong urging action on block B formed within the catheter, the latter recovering its patency by expelling the material of block B from the opening of cavity 14, arranged proximally of the catheter.

In embodiments as exemplified in Figure 1, the material of block B expelled from catheter 10 in a proximal direction may simply flow into syringe S1.

In embodiments as exemplified in Figure 2 and 3, the material of block B expelled from catheter 10 may flow through a vent duct 30, to which a valve 32 may be associated which closes duct 30 when cavity 14 is brought to a subatmospheric pressure level.

In embodiments as exemplified in Figure 1, the action of feeding pressurized fluid to the distal end of cavity 14, performed via duct 22 and syringe S2, may take place while keeping the suction action at the proximal end of cavity 14, performed via syringe S1. In this way, block B formed within the catheter is subjected so to say to a twofold action:
- urging action, in a proximal-distal direction, by the pressurized fluid fed by syringe S2 at the distal end of cavity 14, and
- suction, again in a proximal-distal direction, by the fluid aspirated by syringe S1 at the distal end of cavity 14.

When the catheter has recovered patency, the aspiration operation (for example syringe S1 in Figure 1 or syringe S in the condition of Figure 2) may therefore be continued, until occlusive formation C has been completely removed. If necessary, the previously described operation aiming at restoring the patency of the catheter may be repeated several times, without requiring a catheter replacement.

In various embodiments, if necessary, the occluder element 20 may also be structured as to occlude openings 22a (as schematically shown in Figure 2) when cavity/duct 14 is at the subatmospheric pressure level, so as not to draw fluid from duct 22.

Of course, without prejudice to the underlying principle of the invention, the details and the embodiments may vary, even appreciably, with respect to what has been described by way of non-limiting example only, without departing from the scope of the invention as defined by the annexed claims.

## Claims

1. A catheter for aspirating occlusive formations (C) in a patient's body, including:
- an aspiration duct (14) with a distal suction opening (16), said aspiration duct (14) to be brought to a subatmospheric pressure level (S1, S) to aspirate an occlusive formation (C) through said distal suction opening (16),
- a unblocking duct (22) to feed to the distal portion of said aspiration duct (14) a pressurized fluid (S2, S) to act in a proximal-distal direction on a block (B) formed in said aspiration duct (14), and
- an occluder element (20) to act on said distal suction opening (16) to prevent escape of said pressurized fluid from said distal suction opening (16).

2. The catheter of claim 1, wherein said occluder element (20) is a flap or leaflet valve, preferably brought to closure by said pressurized fluid.

3. The catheter of claim 2, wherein said valve (20) is of a piece with the body of the catheter (10).

4. The catheter of claim 1, wherein said occluder element is an inflatable balloon.

5. The catheter of any of the preceding claims, wherein said unblock duct (22) is a duct having an annular cross section surrounding said aspiration duct (14).

6. The catheter of any of the preceding claims, said catheter having distal end having a chamfered end portion defining said distal suction opening (16).

7. The catheter of any of the preceding claims, comprising a tubular passageway (12) for a guide wire (W) of the catheter.

8. A method of removing a block (B) formed in a catheter for the aspiration of occlusive formations (C) in a patient's body, the catheter comprising an aspiration duct (14) with a distal suction opening (16), said aspiration duct (14) to be brought to a subatmospheric pressure level (S1, S) to aspirate an occlusive formation (C) through said distal suction opening (16), the method including, in the presence of a said block (B) formed in said aspiration duct (14):
- feeding (22) to said distal portion of the aspiration duct (14) a pressurized fluid (S2, S) to act in a proximal-distal direction onto said block (B), and
- preventing (20) the escape of said pressurized fluid from said distal suction opening (16).
